(19) Europäisches Patentamt
European Patent Office
Office européen des brevets

(11) Veröffentlichungsnummer: **0 175 920**
**A2**

(12) # EUROPÄISCHE PATENTANMELDUNG

(21) Anmeldenummer: 85110479.4

(22) Anmeldetag: 21.08.85

(51) Int. Cl.[4]: C 07 C 69/743
C 07 C 67/30, C 07 D 309/30

(30) Priorität: 21.09.84 DE 3434615

(43) Veröffentlichungstag der Anmeldung:
02.04.86 Patentblatt 86/14

(84) Benannte Vertragsstaaten:
CH DE FR GB LI NL

(71) Anmelder: DYNAMIT NOBEL AKTIENGESELLSCHAFT
Postfach 1261
D-5210 Troisdorf, Bez. Köln(DE)

(72) Erfinder: Schmidt, Hans-Georg, Dr.
Porzer Strasse 15
D-5216 Niederkassel 3(DE)

(54) **Verfahren zur Herstellung von Vinylcyclopropancarbonsäurederivaten, dabei einzusetzende Zwischenprodukte und Verfahren zur Herstellung dieser Zwischenprodukte.**

(57) Die vorliegende Erfindung behandelt ein Verfahren zur Herstellung von cis-trans-Isomerengemischen von 2,2-Dimethyl-3(ß,ß-dichlorvinyl)-cyclopropan-1-carbonsäureestern, bei denen das cis-Isomere in bei weitem höherem Anteil entsteht als das trans-Isomere. Das Verfahren geht von Ausgangsverbindungen aus, die bisher nicht zur Herstellung diese Isomerengemischs eingesetzt worden sind und die teilweise auch neue Verbindungen sind.

Das Verfahren ist dadurch gekennzeichnet, daß man entweder ein 4,4-Dimethyl-5-halogen-6-alkoxy-tetrahydropyron oder einen 3,3-Dimethyl-4-halogen-5-oxo-pentansäureester mit Dichlorphosphonsäureestern in Gegenwart von Alkalialkoholaten oder von Alkalihydriden oder von Lithiumalkylen umsetzt. Die als Ausgangsprodukte einsetzbaren Tetrahydropyrone sind neue Verbindungen, für die Stoffschutz und ein Verfahren zu deren Herstellung beansprucht wird. Dieses Herstellverfahren besteht darin, daß ß,ß-Dimethyl-δ, δ -dihalogen- δ -valerolactone mit Alkalialkoholaten umgesetzt werden. Die Reaktionstemperatur beträgt dabei zwischen − 10 und + 50°C.

EP 0 175 920 A2

Troisdorf, den 17.09.1984
OZ 84050 Dr.Sk/br.

DYNAMIT NOBEL AKTIENGESELLSCHAFT
5210 Troisdorf

Verfahren zur Herstellung von Vinylcyclopropancarbonsäurederivaten, dabei einzusetzende Zwischenprodukte und Verfahren zur Herstellung dieser Zwischenprodukte

Gegenstand der vorliegenden Erfindung ist ein neues Verfahren zur Herstellung von Derivaten der Vinylcyclopropancarbonsäure sowie Stoffschutz für die bei diesem Verfahren als Ausgangsprodukte einzusetzenden neuen Derivate des Tetrahydro-α-pyrons. Weiterhin wird ein Verfahren zur Herstellung dieser neuen Derivate des Tetrahydro-α-pyrons beansprucht.

Vinylcyclopropancarbonsäurederivate der im Anspruch 1 genannten Formel I sind wichtige Zwischenprodukte zur Herstellung von Insektiziden aus der Klasse der Pyrethroide. Das Wirkungspotential dieser Pyrethroide hängt u.a. von den stereochemischen Verhältnissen des Wirkungsmoleküls ab. So ist z.B. die cis-Form des Cypermethrins wirksamer als die trans-Form. Die Synthesen des Cypermethrins gehen von 2,2-Dimethyl-3(ß,ß-dichlorvinyl)-cyclopropan-1-carbonsäureestern aus, die im Folgenden auch der Einfachheit halber als Cyclopropancarbonsäureester bezeichnet werden (Synthetic Pyrethroids, ACS-Symposium Series 42, S. 45).

In der EP-B1 0 003 683 wird ein Herstellungsverfahren beschrieben, das die Synthese der cis-Form des genannten Cyclopropancarbonsäureesters zum Ziele hat. Diese Möglichkeit sei hier kurz skizziert:

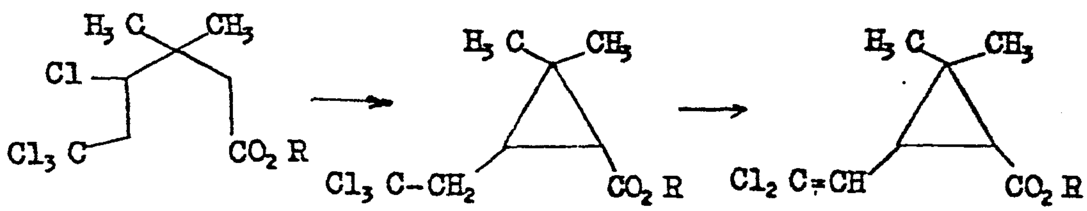

cis Anteil > 50 %   cis Anteil > 50 %

Der Nachteil dieses Verfahrens ist, daß die Reaktion zur cis-Form ganz spezielle Lösungsmittelgemische verlangt und daß die Herstellung des Ausgangsprodukts nur mit hohem technischen Aufwand möglich ist.

Es bestand deshalb die Aufgabe, ein Verfahren zur Herstellung des Cyclopropansäureesters der Formel I aufzufinden, das von anderen als den bisher eingesetzten Ausgangsprodukten ausgehen soll, wobei die Herstellung dieser Ausgangsprodukte nach einfachen Verfahrensweisen durchführbar sein soll und bei dem ein möglichst hoher Anteil an cis-Isomeren entstehen soll.
In Erfüllung dieser Aufgabe wurde nun ein Verfahren zur Herstellung von 2,2-Dimethyl-3(ß,ß-dichlorvinyl)-cyclopropan-1-carbonsäureestern gefunden, das durch die im Anspruch 1 genannten Maßnahmen gekennzeichnet ist.

Bei Anwendung des neuen Verfahrens erhält man ein cis-trans-Isomerengemisch der Verbindung der Formel I

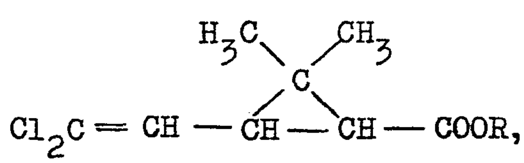

(I)

in der R für einen $C_1$ bis $C_6$-Alkylrest steht, das mehr als 50 % des cis-Isomeren enthält.

Die Ausgangsprodukte für das neue Verfahren sind leicht zugängliche Verbindungen. Die Pentansäureester der Formel II

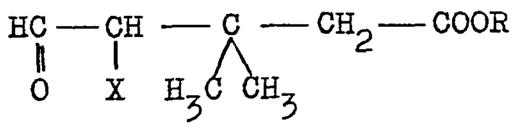

(II)

(R = $C_1$ bis $C_6$-Alkyl, X = Cl oder Br), die auch als 3,3-Dimethyl-4-halogen-5-oxo-pentansäureester bezeichnet werden, sind bereits bekannte Verbindungen; ihre Herstellung erfolgt durch einfache Umsetzung von ß,ß-Dimethyl-γ,δ-dihalogen-δ-valerolactonen mit Alkoholen. Dieses Verfahren wird z.B. in der DE-OS . . . . . (deutsche Patentanmeldung P 34 16 414.6) beschrieben.

Die ebenfalls als Ausgangsverbindungen einsetzbaren 4,4-Dimethyl-5-halogen-6-alkoxy-tetrahydropyrone der im Anspruch 1 genannten Formel III, die auch als ß,ß-Dimethyl-γ-halogen-δ-alkoxy-δ-valerolactone bezeichnet werden können, sind neue Verbindungen. Ihre Herstellung erfolgt ebenfalls aus den obengenannten ß,ß-Dimethyl-γ,δ-dihalogen-δ-valerolactonen der Formel IV

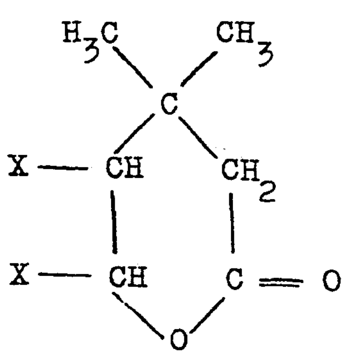

(IV)

(X = Cl oder Br) durch Umsetzung mit Alkalialkoholaten.

Dabei werden die Alkoholate vorzugsweise im stöchiometrischen Verhältnis zu den Valerolactonen eingesetzt, wobei ein geringer Überschuß von 1,1 bis 1,2 : 1 durchaus möglich ist.

Der Alkoholatrest des bei dieser Reaktion einsetzbaren Alkoholats kann bis zu 6 C-Atome besitzen. Vorzugsweise werden die Natrium- und Kaliumalkoholate, beispielsweise die -methylate und -ethylate, verwendet.

Die Herstellung dieser neuen Verbindungen erfolgt im Temperaturbereich zwischen - 10 und + 50° C. Für eine gute Durchmischung der Reaktionspartner ist es empfehlenswert, in einem geeigneten Lösungsmittel zu arbeiten. Als solche sind z.B. möglich: aliphatische und aromatische, bei Raumtemperatur flüssige, Kohlenwasserstoffe, Ether, wie z.B. Tetrahydrofuran, oder Amine, wie z.B. Triethylamin.

Für das erfindungsgemäße Verfahren zur Herstellung der Cyclopropancarbonsäureester der Formel I lassen sich als basische Verbindungen sowohl Alkalialkoholate als auch Alkalihydride als auch Lithiumalkyle einsetzen. Die einzusetzenden Mengen betragen jeweils 1 bis 5 Moläquivalente, vorzugsweise 2 bis 2,5 Moläquivalente basischer Verbindungen, bezogen auf das eingesetzte Ausgangsprodukt.

Als Lithiumalkyle eignen sich dabei solche, deren Alkylgruppe 1 bis 8 C-Atome besitzen, z.B. Butyllithium. Als Alkalihydride eignen sich beispielsweise Natrium- oder Kaliumhydrid. Die bevorzugten basischen Verbindungen sind die Natrium- und Kaliumalkoholate, wobei der Alkoholrest 1 bis 6 C-Atome, vorzugsweise 1 bis 4 C-Atome, haben kann.

Die Chlorphosphonsäureester werden in Mengen von 1 bis 50, vorzugsweise 1 bis 8 Moläquivalenten, bezogen auf die Ausgangsverbindungen, verwendet. Verwendet man kein Lösungsmittel, dann empfiehlt es sich, den Phosphonsäureester mindestens im 5fachen molaren Überschuß einzusetzen, um eine gute Rührfähigkeit des Reaktionsgemisches zu gewährleisten.

Unabhängig davon, welche Ausgangsprodukte für das neue Verfahren zur Herstellung der Cyclopropancarbonsäureester der Formel I eingesetzt werden, kann die Reaktionstemperatur im Bereich von - 50° C bis + 100° C gewählt werden. Überraschend ist dabei, daß auch im oberen Temperaturbereich eine Isomerisierung oder eine direkte Steuerung zum thermodynamisch stabileren trans-Isomeren der allgemeinen Formel I nur teilweise eintritt. Zur Erzielung einer besseren Durchmischbarkeit wird die Reaktion vorzugsweise in einem unter den Reaktionsbedingungen inerten Lösungsmittel durchgeführt. Derartige Lösungsmittel sind beispielsweise Ether, wie z.B. Tetrahydrofuran, 1,4-Dioxan, aromatische Kohlenwasserstoffe, wie z.B. Benzol, Toluol, Chlorbenzol; aliphatische Kohlenwasserstoffe, die geradkettig, verzweigt oder ringförmig sein können, Nitrile, wie z.B. Acetonitril.

Beispiel 1

Zu einer Mischung aus 4,0 g 5-Oxo-4-brom-3,3-dimethylpentansäuremethylester, 3,4 g Dichlormethanphosphonsäuredimethylester und 80 ml der in Tabelle 1 genannten Lösungsmittel werden bei 0° C 2 Moläquivalente der in Tabelle 1 genannten Alkoholate unter Rühren zudosiert. Anschließend wird bei 20° C zwei Stunden gerührt. Nun gibt man 100 ml Eiswasser zu und trennt die organische Phase von der wäßrigen Phase ab. Nach Trocknen der organischen

Phase wird das Lösungsmittel abgezogen und der Rückstand gaschromatographisch und NMR-spektroskopisch untersucht und als 2,2-Dimethyl-3-(ß,ß-dichlorvinyl)-cyclopropancarbonsäuremethylester identifiziert. Das cis-trans-Verhältnis in Abhängigkeit vom Lösungsmittel ist in der Tabelle 1 aufgeführt.

Tabelle 1

| Versuch | Lösungsmittel | Alkoholat | Rohausbeute | cis:trans-Verhältnis |
|---|---|---|---|---|
| a) | Toluol | $NaOCH_3$ | 85 % | 88 : 12 |
| b) | 1,4-Dioxan | $NaOCH_3$ | 80 % | 87 : 13 |
| c) | Acetonitril | $NaOCH_3$ | 83 % | 85 : 15 |
| d) | Toluol | K-tert.-Butylat | 79 % | 90 : 10 |

Beispiel 2

Zu einer Mischung aus 10,0 g 5-Oxo-4-chlor-3,3-dimethylpentansäuremethylester, 21,0 g Dichlormethanphosphonsäuredimethylester und 20 ml Toluol werden bei 0° C 3,0 g $NaOCH_3$ unter Rühren dosiert. Nach einer Stunde werden weitere 3,0 g $NaOCH_3$ dosiert. Nach weiteren drei Stunden Rührzeit bei 10 bis 20° C wird analog zu Beispiel 1 aufgearbeitet. Man erhält 6,7 g 2,2-Dimethyl-3-ß,ß-dichlorvinyl-cyclopropancarbonsäuremethylester. Das Isomerenverhältnis liegt bei cis:trans = 75 : 25.

Beispiel 3

Zu einer Mischung aus 4,0 g 5-Oxo-4-brom-3,3-dimethylpentansäuremethylester, 4,1 g Dichlormethanphosphonsäurediethylester und 56 ml Toluol werden 2,1 g $NaOCH_3$ bei 0° C dosiert. Nach einer Stunde Rührzeit werden weitere 2,1 g $NaOCH_3$ zudosiert. Nach weiteren zwei Stunden Rührzeit bei 10 bis 20° C wird analog zu Beispiel 1 aufgearbeitet. Man erhält 2,6 g einer Mischung aus 2,2-Dimethyl-3-(ß,ß-dichlorvinyl)-cyclopropancarbonsäuremethylester und 2,2-Dimethyl-3-(ß,ß-dichlorvinyl)-cyclopropancarbonsäureethylester. Das Isomerenverhältnis beider Ester liegt bei cis:trans = 95 : 5.

Beispiel 4

Zu einer Mischung aus 5,0 g 4,4-Dimethyl-5-brom-6-methoxytetrahydro-α-pyron, 4,5 g Dichlormethanphosphonsäuredimethylester und 70 ml Toluol werden 2,4 g $NaOCH_3$ bei 5° C dosiert. Nach einer Stunde Rührzeit werden weitere 2,4 g $NaOCH_3$ zudosiert und anschließend zwei Stunden bei 10 bis 20° C gerührt. Danach wird analog Beispiel 1 aufgearbeitet. Man erhält 3,3 g 2,2-Dimethyl-3-(ß,ß-dichlorvinyl)-cyclopropancarbonsäuremethylester. Das Isomerenverhältnis beträgt cis:trans = 97 : 3.

Beispiel 5

10 g 4,4-Dimethyl-5,6-dichlor-tetrahydro-α-pyron werden in 30 g Triethylamin gelöst und zu dieser Mischung 2,7 g $NaOCH_3$ unter Rühren bei 20° C zudosiert. Danach wird vier Stunden bei 20° C gerührt und der Feststoff abfiltriert. Das resultierende Filtrat wird destilliert. Bei 100 bis 110° C/0,2 Torr destillieren 7,1 g 4,4-Dimethyl-5-chlor-6-methoxy-tetrahydro-α-pyron in Form eines cis-trans-Isomerengemisches (cis:trans = ca. 20 : 80).

$^1$H-NMR-Spektrum (90 MHZ, $CDCl_3$) $\delta$ ppm: 1,12 (3 H); 1,15 (3 H); 1,19 (3 H)*; 1,22 (3 H)*; 2,47 (2 H); 2,68 (2 H)*; 3,60 (3 H); 3,63 (3 H)*; 3,78 (1 H); 3,98 (1 H)*; 5,17 (1 H); 5,31 (1 H)*.

Die mit * bezeichneten Signale werden dem cis-Isomeren zugeordnet.

Beispiel 6

Analog zu Beispiel 5 wird aus 4,4-Dimethyl-5,6-dibrom-tetrahydro-$\alpha$-pyron das 4,4-Dimethyl-5-brom-6-methoxy-tetrahydro-$\alpha$-pyron hergestellt. Sdp.: 90 bis 95$^0$ C/ 0,3 Torr. Isomerenverhältnis cis:trans = ca. 10 : 90.

$^1$H-NMR-Spektrum (30 MHZ, $CCl_4$) $\delta$ ppm = 1,25 (6 H); 2,60 (2 H); 3,65 (3 H); 4,0 (1 H); 5,35 (1 H).

Troisdorf, den 17.09.1984
OZ 84050 Dr.Sk/br.

## Patentansprüche

1. Verfahren zur Herstellung von cis-trans-Isomerengemischen von 2,2-Dimethyl-3(ß,ß-dichlorvinyl)-cyclopropan-1-carbonsäureestern der Formel I, die mehr als 50 % des cis-Isomeren enthalten,

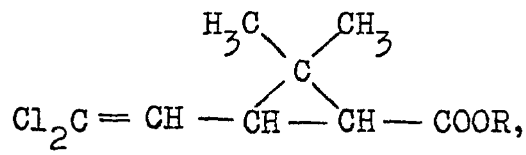

(I)

in der R für einen Alkylrest mit 1 bis 6 C-Atomen steht, dadurch gekennzeichnet, daß man entweder

a) einen Pentansäureester der Formel II

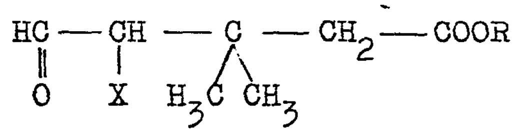

(II)

,

in der X für Chlor oder Brom steht und R die obengenannte Bedeutung hat

oder

b) ein 4,4-Dimethyl-5-halogen-6-alkoxy-tetrahydropyron der Formel III

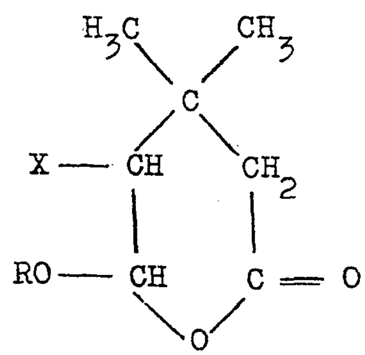

(III)

,

in der X und R die obengenannte Bedeutung haben,

mit einer basischen Verbindung in Gegenwart eines Dichlorphosphonsäureesters der Formel

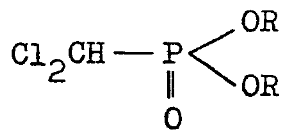

,

in der R die genannte Bedeutung hat, umsetzt.

2. Verfahren gemäß Anspruch 1, dadurch gekennzeichnet, daß man als basische Verbindung ein Alkalialkoholat einsetzt.

3. Verfahren gemäß Anspruch 1, dadurch gekennzeichnet, daß man als basische Verbindung ein Alkalihydrid einsetzt.

4. Verfahren gemäß Anspruch 1, dadurch gekennzeichnet, daß man als basische Verbindung ein Lithiumalkyl einsetzt.

5. Verfahren gemäß einem der Ansprüche 1 bis 4, dadurch gekennzeichnet, daß die Umsetzung in einem Lösungsmittel durchgeführt wird.

6. 4,4-Dimethyl-5-halogen-6-alkoxy-tetrahydropyrone der Formel III

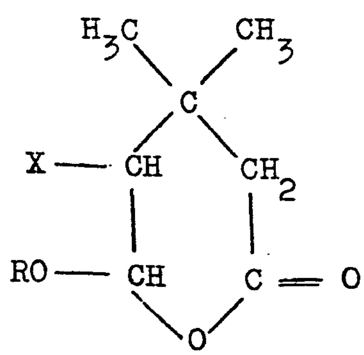

(III)

,

in der X für Chlor oder Brom und R für einen Alkylrest mit 1 bis 6 C-Atomen stehen.

7. Verfahren zur Herstellung von 4,4-Dimethyl-5-halogen-6-alkoxy-tetrahydropyronen der im Anspruch 6 genannten Formel III, dadurch gekennzeichnet, daß man ein 4,4-Dimethyl-5,6-dihalogen-tetrahydro-δ-pyron mit einem Alkalialkoholat zur Reaktion bringt.